# EUROPEAN PATENT SPECIFICATION

(11) **EP 2 218 396 B1**
(45) Date of publication and mention of the grant of the patent: **10.12.2014**
(21) Application number: 10153008.7
(22) Date of filing: 09.02.2010
(51) Int. Cl.: A61B 5/00, A61B 19/00

(54) **Robotic manipulator for the remote maneuvering of catheters**
Robotische Manipulator zum ferngesteuerten Maneuvrieren von Kathetern
Manipulateur robotique pour manoeuvrer à distance des cathéters

(30) Priority: 11.02.2009 IT BO20090004 U
(43) Date of publication of application: 18.08.2010
(73) Proprietor: Tre Esse Progettazione Biomedica S.r.l, 40138 Bologna (IT)
(72) Inventor: Plicchi, Gianni, I-40125, BOLOGNA (IT); Marcelli, Emanuela, I-40125, BOLOGNA (IT); Cercenelli, Laura, I-60030, CASTELBELLINO, Province of Ancona (IT); Panfili, Mauro, I-40139, BOLOGNA (IT)
(74) Representative: Porsia, Attilio

(56) References cited:
- US-A- 4 753 248
- US-A1- 2001 021 841
- US-A1- 2002 177 789
- US-A1- 2005 043 618

## Description

The invention relates to a robotic manipulator for the remote manoeuvring, in the human cardiovascular system, of temporary catheters for electrophysiology procedures, these catheters being provided with controls for deflection of the distal end and therefore being described as "steerable". More specifically, the invention relates to constructional improvements to the robotic manipulator described in Italian patent application B02005A-799 in the name of the present applicant, to which the most general reference will be made, and which describes an apparatus for this purpose, of the type shown schematically in the attached Figure 1, which uses an introducer A which is also protected by Italian patent application BO2005A-798 in the name of the present applicant, and which comprises a said robotic manipulator B to be placed near the patient P, in a relatively fixed and predetermined position, for supporting and guiding the catheter C by its handle D, the manipulator comprising telescopic stiffening means E, with which the portion of said catheter which lies outside the patient's body, and which lies between said handle and said introducer and is gripped by said telescopic means E, is kept axially aligned with said handle and essentially rigid, such that it cannot bend as a result of the buckling force when the catheter is manoeuvred by the robotic manipulator via the handle. The robotic manipulator B is designed to support the handle D and the slider F or other suitable means for deflecting the distal end of the catheter and is remotely controllable by an operator in a remote position who is shielded from the ionizing radiation emitted by the system which displays the navigation of said catheter in the human cardiovascular system. For operating the catheter, the robotic manipulator B must be capable of subjecting the catheter, by supporting its handle, to advance and retraction movements, to axial rotation in both directions, and to the necessary steering movements to enable the tip of the catheter to be steered through the twists and turns of the cardiovascular system of the patient P and to reach a predetermined point of this system, in a controlled, safe and repeatable way.

The robotic manipulator described in the patent application to which reference is made comprises a body B1 which is associated, by means of a rectilinear guide and sliding block unit G having a screw and nut actuator and a geared motor and encoder, with the end of a hinged and/or jointed arm H, having rapidly and easily connectable and disconnectable hinges and/or joints, by means of which the guide of said system G can be fixed with the desired orientation for a desired position of the bed L and/or other structure supporting the patient. The body B1 of the manipulator carries at its end and in axial alignment a shaft which can be rotated in a precise way by means of a geared motor with encoder positioned in the body B1, and an arm B2 with an L-shaped lateral profile is fixed to this shaft by its base so that it projects therefrom, this arm forming a longitudinal extension of the said body of the manipulator. On the arm B2, means M are provided to fix the handle D of the catheter longitudinally, and gripping means N are provided to act on the slider F, push button or other means of this handle which is connected to the catheter steering means, and said gripping means N are connected to an actuator, of the rectilinear motion type for example, with a corresponding geared motor and encoder, housed inside B2. The means which support the catheter handle are mounted on a guide and sliding block unit connected to a load sensor which generates an electrical signal proportional to the resistance encountered by the handle in the axial movement of the catheter, for the purpose of detecting whether or not the resistance encountered by the catheter is acceptable during the movements imparted by the operator's remote control actions. All the electrical and electronic components in the terminal arm of the manipulator which supports the catheter handle are connected to a terminal block fixed in the body B1 of the manipulator, by means of flexible electrical cables which pass through curved slots which have a substantial angular amplitude and which are formed in the facing bases of the arm B2 and the body B1.

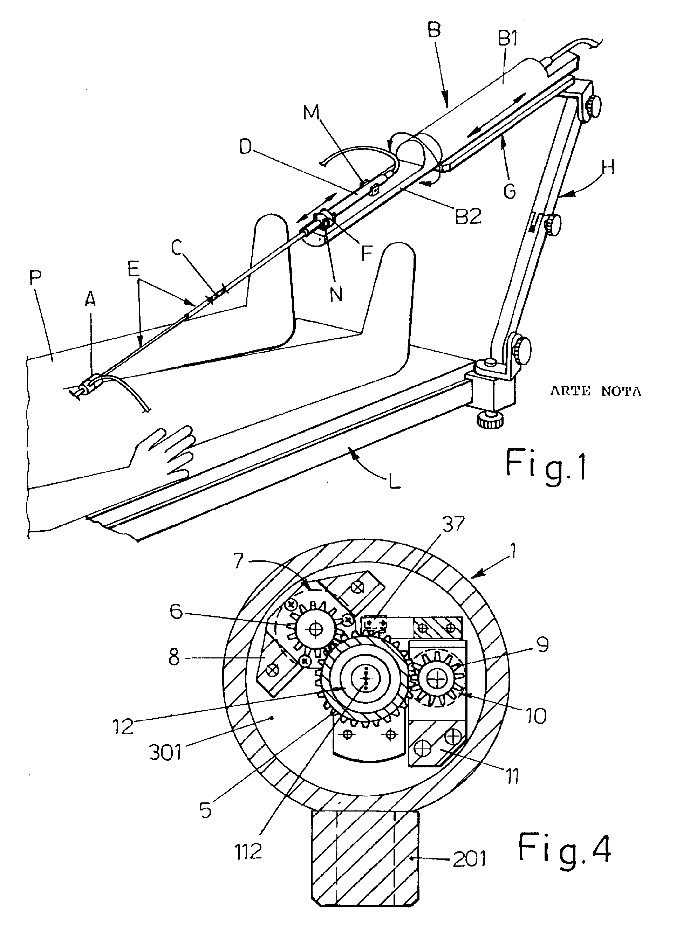

Tests of a manipulator constructed in this way have revealed the following limitations. A first limitation is due to the limited and difficult axial rotation of the arm B2 of the manipulator, caused by said curved slots, and the invention proposes to overcome this limitation by using a rotary collector positioned axially in the manipulator body. A second limitation arises from the considerable length of the manipulator when in the maximally extended state, this being due to the fact that the catheter advance and retraction means G are located on the body B1 of the manipulator of Figure 1. The invention proposes to overcome this limitation by providing the catheter advance and retraction means on the arm B2 which also supports the catheter handle and has a length related to that of the handle, and which is therefore adapted for this use.

The body B1 of the manipulator can thus be made with limited dimensions in length and particularly can be mounted on a static supporting structure H, with advantages of constructive and reliability simplification in the operation of the rotation and control means arranged at the interior of said body B1, inasmuch as said means can be connected to fixed electric cables and not to movable electric cables as in the embodiment of Figure 1 (see beyond).

Another limitation of the known solution arises from the lower degree of safety that might be encountered as a result of having the movement encoders connected directly and solely to the electric motors of the three actuators used. The invention proposes to overcome this limitation by using supplementary encoders to detect the motion of the terminal part of the kinematic chain driven by each of said motors.

As additional prior art there can be cited the following documents which resulted from the European Search Report issued in connection with the present application and considered in the attached search opinion.
- US 2002/177789, identified as D1;
- US 2005/043618, identified as D2,
- US 4 753 248, identified as D3.

Document D1 having as title "System and methods for advancing a catheter" discloses a robotic manipulator for the insertion and retraction a catheter into and from the human body, for example for cardiac diagnostics. This document teaches exclusively how to impart to a catheter advancement and retraction movements and not also rotational movements to the right and to the left, by driving it from a fixed point. The manipulator described in this document is not apt for the use in catheters of the (steerable) steering or similar types, in order to obtain that the catheter is caused to rotate and steer in the sinuosity of the cardiovascular system of the patient and can reach the predetermined point of said system in a controlled safe an repeatable manner. The same document does not teach how to detect anomalous loads on the tip of the catheter in the insertion phase into the patient's body, so as to avoid very dangerous perforations of the cardiovascular system.

Document D2 having as title "Automatic/manual longitudinal position translator and rotary drive system for catheters" describes a robotic manipulator for imparting to a catheter movements of advancement and retraction and of left-right rotation. This document does not teach how to impart said composite movements to the unit of a catheter comprising the handle and particularly to catheters of the steering type. According to this document the means for the rotation of the catheter are mounted on the chassis which provides to the translation of the catheter itself, so that these means are governed with difficulty and scarce reliability, from a fixed point. Also this document foes not teach how to detect anomalous loads on the tip of the catheter in the insertion phase into the patient's body, so as to avoid very dangerous perforations of the cardiovascular system.

Document D3 having as title "Probe translation system for the use in hyperthermia treatment" describes a robotic apparatus adapted to impart only axial movements of advancement and retraction to a catheter in hyperthermia treatment. The manipulator described in this document is not apt for the use in catheters of the steering or similar types, in order to obtain that the catheter is caused to rotate and steer in the sinuosity of the cardiovascular system of the patient , and can reach the predetermined point of said system in a controlled safe an repeatable manner. The same document does not teach how to detect anomalous loads on the tip of the catheter in the insertion phase into the patient's body, so as to avoid very dangerous perforations of the cardiovascular system.

These and other characteristics of the invention, and the advantages deriving therefrom, are illustrated more fully in the following description which refers to the figures on the three attached sheets of drawing, in which, in addition to Figure 1 which has already been discussed:
- Fig. 2 is a lateral view, with parts in longitudinal section, of the new robotic manipulator, with its components positioned in the start of cycle condition;
- Fig. 3 is a plan view from above of the manipulator with the catheter handle support arm, with the covering elements removed in order to reveal the internal components, and without the means which acts on the catheter steering controller;
- Figs. 4, 5 and 6 show three details of the construction of the manipulator, taken, respectively, along the section lines IV-IV, V-V and VI-VI of the preceding Figures 2 and 3.

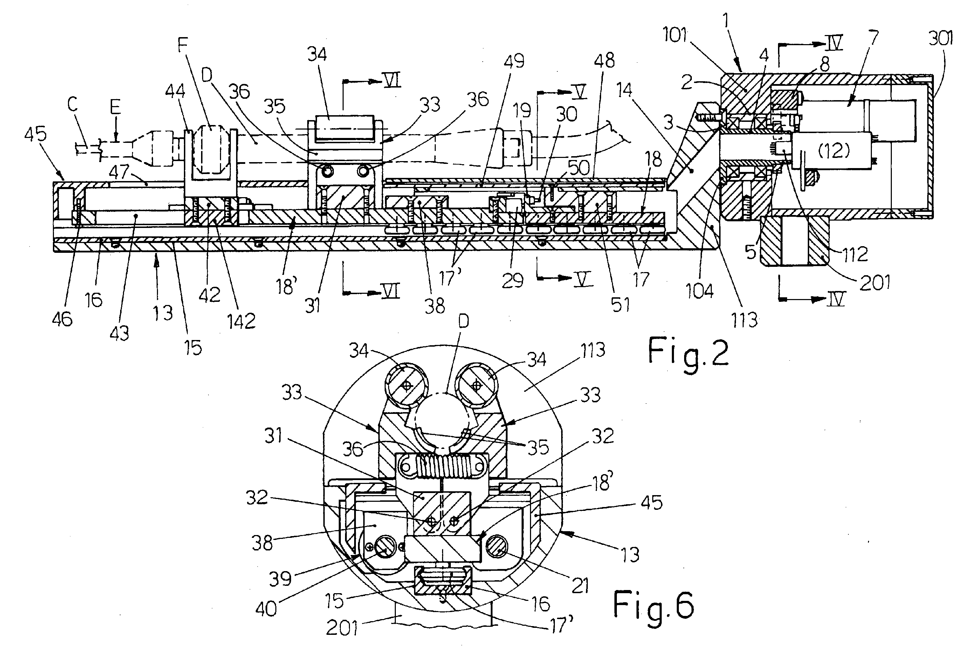

Figures 2, 3 and 4 show that the manipulator comprises a boxlike body 1, of cylindrical shape for example, with a round cross section, having a base 101 of considerable thickness and having a lateral appendage 201 for connection to the hinged and/or jointed arm for supporting the manipulator in operation, as indicated for example by H in Figure 1, the body being closed at its rear with an end plate 301 which is removable for access to the internal parts of the body 1, and having apertures which are not shown for the passage of the electrical power supply and control cables of the manipulator. The end plate 101 is provided with an axial hole 2 in which is rotatably housed, by means of bearings and/or other suitable means 3, a hollow shaft 4 which has an external flange 104 and which has a portion, projecting into the body 1, on which is keyed a ring gear 5 which engages with a pinion 6 keyed on the axle of a reversible geared motor unit 7 with an encoder, fixed by flanges to a support 8 fixed to the end plate 101, said ring gear 5 also engaging with a pinion 9 keyed on the axle of an encoder 10 fixed to the same end plate 101 by means of a support 11. The encoder 10 generates an electrical signal which is processed in combination with that received from the encoder of the drive unit 7, to detect any malfunction of the kinematic chain in which it is fitted, even if the encoder of the unit 7 detects normal operation, for example if there is any slippage of one of the two components of the gearing 5, 9. In Figure 4, the number 37 indicates a microswitch which can read a projecting or recessed part of the hollow shaft 4, close to the ring gear 5, and which provides the "zero" reference for the computing system with which said safety encoder 10 and the encoder of the drive unit 7 are associated.

The rotating element 112 of a rotary collector 12 is fitted partially into the end of the hollow shaft 4 which projects into the body 1 (Fig. 2), in a substantially coaxial arrangement, to enable fixed cables (not shown) which enter the body 1 to be connected to all the power supply and control circuits of the electrical and electronic components of the manipulator located on the arm of the manipulator, which is fixed to the flange 104 and which supports the catheter handle as described below.

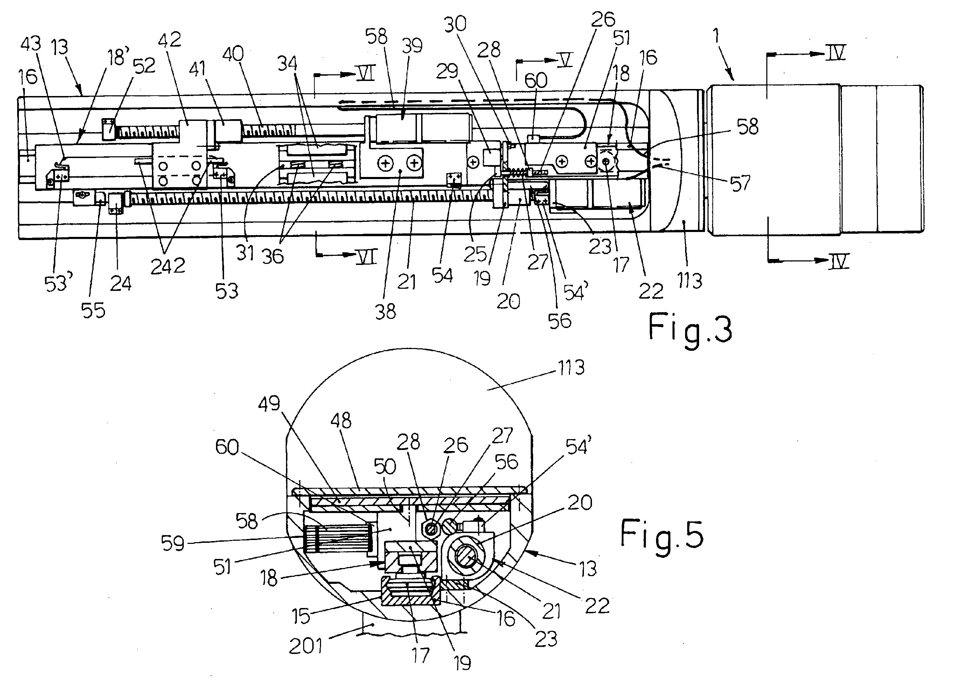

The flange 104 of the hollow shaft 4 (Figs. 2 and 3) is fixed to the base 113 of the L-shaped arm 13, which is similar in shape and dimensions to the arm B2 of Figure 1 but differs from the latter in that it is essentially composed of two parts fitted telescopically into each other, enabling the axial advance and retraction movement to be imparted to the catheter in addition to the steering movement. All the electrical parts that operate in the arm 13 are connected to the rotating element 112 of the collector 12 with electrical cables (see below) which pass through an inclined hole 14 formed in the base 113 of this arm and which pass through the hollow shaft 4, as shown in Figure 2. Figures 5 and 6 show that the body of the arm 13 has a substantially semicircular cross section and carries on its base, along its whole length, a longitudinal channel 15 in which is fixed a C-section guide 16, on the inner sides of which the rollers 17, 17' of two flat carriages 18, 18' run and bear alternately, these rollers having different lengths and being positioned one after another. The first of the carriages 18, which is shorter and is close to the base 113 of the arm 13, has a cross-piece 19 which is fixed at its front and supports a nut 20 interacting with a screw 21 which is positioned laterally and parallel to said guide 16 and which has its end facing said base 113 keyed to the slow output shaft of a reversible geared motor unit 22 with an encoder, fixed by flanges to a support 23 fixed inside the arm 13. On the free end of the screw 21 there is keyed an encoder 24 which generates a signal correlated with the rotation of the screw, this signal being processed in combination with that which is produced by the encoder of the motor 22 in order to detect any malfunction of the means for the longitudinal movement of the carriage 18. The longer carriage 18' is provided, on the end close to the short carriage 18, with an appendage 25 which extends partially in front of the cross-piece 19 and to which a small screw 26 is fixed parallel to the screw 21 with a stud, this small screw passing through a hole in the cross-piece 19 with a degree of clearance and having a cylindrical helical spring 27, loaded by a nut 28, fitted on the part which projects rearwards from the cross-piece. As a result of the compression of said spring 27, the moving element of a force sensor 29 fixed to the front carriage 18' touches part of the cross-piece 19, is suitably pre-loaded by the calibration of said spring, and is in the optimal condition for generating an electrical signal correlated with the resistance encountered by the catheter in the phase of advance into the patient's cardiovascular system, since the handle of the catheter is associated with the front carriage 18' as described below. In Figures 2 and 3, the number 30 indicates schematically a screw and nut assembly, with the screw fixed to the carriage 18', which passes with a degree of clearance through a hole formed in the cross-piece 19 and which carries the nut laterally with respect to the latter. This assembly has the function of providing a sufficient travel for bringing the carriages 18, 18' towards each other, for the operation of the force sensor 29, and of coupling the carriages 18 and 18' together during the return travel of the two carriages. In the intermediate part of the front carriage 18' there is fixed in a central position a body 31 on the opposite ends of which the lower ends of H-shaped jaws 33 are pivoted at 32, these jaws having upper limbs which rotatably support rubber-coated rollers 34 which are parallel to each other, the jaws also having intermediate parts of curved shape with anti-friction shoes 35 and being pressed against each other by return springs 36. The handle D of the catheter (Figs. 2 and 6) can be inserted and fixed between the rollers 34 and said shoes 35 in such a way that it is rapidly removable when necessary, this handle being firmly secured to the carriage 18' and in axial alignment with the hollow shaft 4, as a result of the appropriate design of the parts which have been described, to ensure that the rotations of this shaft are transmitted to the catheter without undesired translational components.

On the front carriage 18', upstream of the aforesaid clamp 31-36, there is fixed a support 38 which has a lateral appendage supporting the reversible geared motor unit 39 with an encoder, which drives a screw 40 which is parallel to the carriage 18' and which interacts with a nut 41 fixed to a sliding block 42 which, by means of its lower longitudinal extension 142, slides in a guided way in a longitudinal median slot 43 of the front carriage 18'. A fork or other suitable gripping device 44, similar to that indicated by N in Figure 1, is fixed to the sliding block 42 and interacts with the slider or other suitable means F provided on the catheter handle D, to actuate the catheter steering control. A cover 45 with a cross section in the form of an inverted U is fixed to the front carriage 18' at 46, runs inside the body of the arm 13 of the manipulator (Figs. 2 and 6) and has a longitudinal slot 47 through which said gripping device 44 passes. During the extension travel of the carriages 18 and 18' for the longitudinal axial advance movement of the catheter, the upper part of the manipulator arm 13 remains covered by an upper cover 48 fixed to the arm and by lower covers 49 fixed at 50 to a support 51 fixed to the rear carriage 18 (Figs. 2 and 5). It will be clear that, as shown in Figure 3 for example, the rotation of the screw 21 results in the longitudinal advance or retraction movement of the catheter and the rotation of the screw 40 results in the steering movement of the catheter. The screw 40 is also connected at its free end to an encoder 52 fixed in the arm 13, the signal from which is processed in combination with that produced by the encoder of the drive unit 39, to detect any malfunctions of this kinematic chain. In Figure 3, the numbers 53 and 53' indicate microswitches which interact with opposing appendages 242 of the sliding block 42 to generate end-of-travel signals or commands for this sliding block. Similar microswitches are provided to generate end-of-travel signals or commands for the carriages 18 and 18', as indicated by the microswitch 54 positioned on board the carriage 18', which interacts with the end-of-travel switch 55 fixed to the arm 13 and with the microswitch 54' fixed to an appendage of the support 23, which interacts with a push rod 56 fixed perpendicularly to the cross-piece 19. The electrical commands sent from the various microswitches and encoders and the power supplies to the motors of the units 22 and 39 travel along electrical cables as indicated for example by 57 and 58 in Figure 2, the latter of which is, for example, in the form of a flexible multi-wire strip partially housed in a recess 59 of an inner side of the arm 13, and is connected to a terminal block 60 fixed laterally to said support 51 (Figs. 2, 3 and 5). The manipulator which has been described is adapted to be enclosed in a sterilized plastic bag with a length matching the extended dimension of the manipulator, by means of which the manipulator can be fixed for operation in the requisite sterile conditions. Clearly, the manipulator which has been described can be modified in its construction in all of those ways which offer equal utility and which are based on the same inventive concept, without departure from the guiding principle of the present invention as described and illustrated herein and as claimed below.

## Claims

1. Robotic manipulator for the remote maneuvering of steerable catheters (C) in the human cardiovascular system, of the type for subjecting the catheter, by supporting its handle (D)and by stiffening its outer portion by means of a telescopic guide (E), to axial advance and retraction movements, to axial rotation in both directions, and to the necessary steering movements to enable the tip of the catheter to be steered through the twists and turns of the cardiovascular system of the patient (P) and to reach a predetermined point in this system, in a controlled, repeatable and safe way, the handle (D) being provided with a slider (F) suitable for the catheter steering control, comprising:
a) a body (1) able to be fixed to adjustable support means (H) with correct positioning and orientation with respect to the patient;
b) an arm (13) fixed in a projecting way and rotatably about a shaft (4) of said body (1), in which body the means for producing said rotation are housed;
c) at least one carriage (18') being mounted in said arm (13) in such a way that it can travel longitudinally, in at least one suitable guide (16), the carriage having an intermediate clamp (31-36) for supporting the handle (D) of the catheter (C) which is axially aligned with said axis of rotation for imparting to the catheter the desired movement of axial rotation, said handle (D) being firmly clamped to said carriage (18') and axially aligned with said shaft (4) such that the rotations of this shaft (4) are transmitted to the catheter (C) without undesired translational components;
d) said carriage (18') being movable by drive means positioned in the arm (13) to produce the advancement and retraction of the catheter in the cardiovascular system;
e) a sliding block (42) being mounted on said carriage (18') with a gripping device (44) acting on the slider (F) provided for the catheter steering control, this sliding block being actuated by drive means associated with the carriage (18');
f) wherein the rotating shaft (4) which carries said arm (13) is of the hollow type and abuts, at one end, a rotary collector (12) whose stator is fixed in said body (1) of the manipulator, while the other end of said hollow shaft abuts a channel (14) formed in the base (113) of said arm (13), through which pass all the electrical cables (57, 58) connected to all the electrical and electronic components provided in the arm, these cables being connected by means of said collector to fixed cables which are also partially connected to various electrical components positioned in the body for rotating said arm (13).

2. Robotic manipulator according to Claim 1, **characterized in that** it comprises a front carriage (18') and a rear carriage (18), the drive means which impart the longitudinal advance and retraction movement to said carriage (18') acting on the rear carriage (18) which is connected to the front carriage (18') by connecting means (26, 28) which comprise resilient and preferably adjustable means (27) which provide correct pre-loading of a force sensor (29) fixed to the front carriage (18'), this sensor generating an electrical signal proportional to the tip force to which the catheter (C) is subjected while being advanced by the manipulator in the human cardiovascular system.

3. Robotic manipulator according to Claim 2, in which said resilient and preferably adjustable means comprise a spring (27) and in which stop means (30) are provided to provide the mutual coupling between said carriages (18, 18') during their retraction travel, in order to avoid stressing said spring (27) for pre-loading said force sensor (29).

4. Robotic manipulator according to any one or more of the preceding claims, in which the drive means for the longitudinal movement of said two carriages (18, 18') and of said sliding block (42) with the steering control comprise two kinematic chains, in which, preferably, end-of-travel microswitches (54, 54', 53, 53') also operate, screw and nut units (21-20, 40-41) and reversible geared motor units (22, 39) with respective encoders, corresponding encoders (24, 52) being provided directly on the screws themselves, the electrical signals from which are processed in combination with those from the encoders of said drive units in order to identify any malfunctions of the two kinematic chains..

5. Robotic manipulator according to any one or more of the preceding claims, in which the arm (13) which carries the catheter handle is made to rotate by a kinematic chain comprising a reversible geared motor unit (7) with an encoder, which is fixed in said body (1) of the manipulator and which, by means of a pinion (6), drives a ring gear (5) keyed on the shaft (4) which rotatably supports said arm, this ring gear (5) being made to engage with a second pinion (9) which drives a further encoder (10), whose electrical signal is processed in combination with that of the encoder of said motor (7) in order to identify any malfunctions of this kinematic chain which also includes means (37) for producing an electrical signal corresponding to a zero or end-of-travel position of this kinematic chain.

6. Robotic manipulator according to any one or more of the preceding claims, **characterized in that** it comprises a self-centering resilient clamp (31-36) for supporting the handle (D) of the catheter while permitting rapid and easy mounting and removal.

7. Robotic manipulator according to any one or more of the preceding claims, **characterized in that** it comprises covers (48, 49) which are partly fixed and partly movable and **in that** the arm (13) comprises a hollow part, said covers being able to keep the hollow part of the arm (13) constantly closed off from the outside while said composite carriage (18, 18'), with the associated parts for supporting, moving and driving the handle (D) of the catheter (C), is travelling in the arm.

## Patentansprüche

1. Ein robotischer Manipulator zum ferngesteuerten Manövrieren von lenkbaren Kathetern (C) im menschlichen kardiovaskulären System, des Typs, auf den durch Halten seines Griffs (D) und Versteifung seines äußeren Teils mit Hilfe einer teleskopischen Führung (E) axiale Vorwärts- und Rückwärtsbewegungen, axiale Umdrehung in beiden Richtungen und die Steuerbewegungen übertragen werden, welche notwendig sind, um die Spitze des Katheters in die Lage zu versetzen, durch die Windungen und Drehungen des kardiovaskulären Systems des Patienten (P) zu steuern und einen vorbestimmten Punkt in diesem System zu erreichen, und zwar in kontrollierter, wiederholbarer und sicherer Weise, wobei der Griff (D) mit einem Schieber (F) ausgerüstet ist, der für die Kontrolle der Kathetersteuerung geeignet ist, welcher jeweils folgendes umfasst:
a) einen Körper (1), der an einstellbaren Tragelementen (H) mit korrekter Positionierung und Ausrichtung im Verhältnis zum Patienten befestigt werden kann;
b) einen Arm (13), welcher hervorstehend und um einen Schaft (4) des genannten Körpers (1) drehbar befestigt ist, in dessen Körper die Elemente, die die jeweilige Umdrehung verursachen, untergebracht sind;
c) mindestens einen Wagen (18`), welcher in diesem Arm (13) so montiert ist, dass er sich längs in mindestens einer entsprechenden Führung (16) bewegen kann, wobei der Wagen eine Zwischenarretierung (31-36) zur Halterung des Griffs (D) des Katheters (C) aufweist, welcher jeweils axial mit der genannten Umdrehungsachse ausgerichtet ist, um dem Katheter die gewünschte Bewegung der axialen Umdrehung zu verleihen, wobei der genannte Griff (D) fest am genannten Wagen (18`) verriegelt und axial mit dem genannten Schaft (4) ausgerichtet ist, so dass die Umdrehungen dieses Schafts (4) auf den Katheter (C) jeweils ohne unerwünschte Verschiebungskomponenten übertragen werden;
d) wobei dieser Wagen (18`) durch entsprechende Antriebselemente bewegt werden kann, welche sich im Arm (13) befinden, um die jeweilige Vor- und Rückwärtsbewegung des Katheters im kardiovaskulären System zu erzeugen;
e) wobei ein Gleitblock (42) auf diesen Wagen (18`) mit einer Greifvorrichtung (44) montiert ist, der auf dem Schieber (F) funktioniert, welcher für die Kontrolle der Kathetersteuerung vorgesehen ist, und dieser Gleitblock durch Antriebselemente betätigt wird, die mit dem Wagen (28') verbunden sind;
f) wobei der Umdrehungsschaft (4), welcher den genannten Arm (13) trägt, hohl ist und an einem Ende an einen Drehkollektor (12) grenzt, dessen Stator in dem genannten Körper (1) des Manipulators befestigt ist, während das andere Ende des genannten hohlen Schaft jeweils an einen Kanal (14) grenzt, welcher an der Basis (113) des genannten Arms (13) gebildet wird, und durch den alle Stromkabel (57, 58) führen, die jeweils mit allen elektrischen und elektronischen Bauteilen, welche in dem Arm vorgesehen sind, verbunden sind, wobei diese Kabel jeweils mit Hilfe des genannten Kollektors mit entsprechenden festen Kabeln verbunden sind, die ebenfalls teilweise jeweils mit verschiedenen elektrischen Komponenten verbunden sind, welche sich im Körper zwecks Umdrehung des genannten Arms (13) befinden.

2. Ein robotischer Manipulator gemäß Anspruch 1, **dadurch gekennzeichnet, dass** er einen vorderen Wagen (18`) und einen hinteren Wagen (18) umfasst, wobei die Antriebsmittel, welche die Längsvor- und -Rückwärtsbewegung auf den genannten Wagen (18`) übertragen, auf den hinteren Wagen (18) einwirken, welcher mit dem vorderen Wagen (18`) durch entsprechende Verbindungselemente (26, 28) verbunden ist, die federnde und vorzugsweise einstellbare Elemente (27) umfassen, welche für die korrekte Vorspannung eines Kraftsensors (29) sorgen, der am vorderen Wagen (18') befestigt ist, wobei dieser Sensor jeweils ein elektrisches Signal erzeugt, welches sich proportional zu der Spitzenstärke verhält, der der Katheter (C) ausgesetzt ist, währen er durch den Manipulator in das menschliche kardiovaskuläre System vorgeschoben wird.

3. Ein robotischer Manipulator gemäß Anspruch 2, bei dem die genannten federnden und vorzugsweise einstellbaren Elemente eine Feder (27) umfassen und bei dem entsprechende Arretierungselemente (30) vorgesehen sind, um für die gegenseitige Verkoppelung zwischen den genannten Wagen (18, 18') während deren Rückwärtsbewegung zu sorgen, damit eine Beanspruchung der genannten Feder (27) zur Vorspannung des genannten Kraftsensors vermieden wird.

4. Ein robotischer Manipulator gemäß einem beliebigen oder mehreren der vorausgegangenen Ansprüche, bei dem die Antriebselemente für die Längsbewegung der genannten beiden Wagen (18, 18') und des genannten Gleitblocks (42) mit der Steuerkontrolle zwei kinematische Ketten umfassen, an denen vorzugsweise auch Anschlags-Mikroschalter (54, 54', 53, 53') funktionieren, Schrauben- und Muttereinheiten (21-20, 40-41) und umsteuerbare Getriebemotoreinheiten (22, 39), mit den jeweiligen Encodern, wobei die entsprechenden Encoder (24, 52) direkt auf den Schrauben selbst vorgesehen sind, und deren elektrische Signale jeweils zusammen mit denen von den Encodern der genannten Antriebseinheiten verarbeitet werden, um jede beliebige Fehlfunktion der beiden kinematischen Ketten feststellen zu können.

5. Ein robotischer Manipulator gemäß einem beliebigen oder mehreren der vorausgegangenen Ansprüche, bei dem der Arm (13), welcher den Kathetergriff trägt, so beschaffen ist, dass er durch eine kinematische Kette zum Drehen gebracht wird, die eine umsteuerbare Getriebemotoreinheit (7) mit einem Encoder umfasst, der an dem genannten Körper (1) des Manipulators befestigt ist und der mit Hilfe eines Getrieberads (6) einen entsprechenden Zahnkranz (5) antreibt" welcher auf dem Schaft (4) verkeilt ist, der den genannten Arm drehbar trägt, wobei dieser Zahnkranz (5) so beschaffen ist, dass er in ein zweites Getrieberad (9) einrastet, das einen weiteren Encoder (10) antreibt, dessen elektrisches Signal jeweils zusammen mit dem des Encoders des genannten Motors (7) verarbeitet wird, um jede beliebige Fehlfunktion dieser kinematischen Kette feststellen zu können, welche ebenfalls Elemente (37) zur Erzeugung eines elektrischen Signals umfasst, das einer Null oder der Anschlagsstellung dieser kinematischen Kette entspricht.

6. Ein robotischer Manipulator gemäß einem beliebigen oder mehreren der vorausgegangenen Ansprüche, **dadurch gekennzeichnet, dass** er eine selbstzentrierende federnde Klammer (31-36) zur Halterung des Griffs (D) des Katheters umfasst, bei gleichzeitiger Ermöglichung einer schnellen und einfachen Montage und Entfernung.

7. Ein robotischer Manipulator gemäß einem beliebigen oder mehreren der vorausgegangenen Ansprüche, **dadurch gekennzeichnet, dass** er Abdeckungen (48, 49) umfasst, welche jeweils teilweise fest und teilweise beweglich sind, sowie dadurch, dass der Arm (13) einen hohlen Teil umfasst, wobei die genannten Abdeckungen in der Lage sind, den hohlen Teil des Arms (13) ständig von der Außenseite abzuriegeln, während der genannte zusammengesetzte Wagen (18, 18`) mit den entsprechenden dazu gehörigen Teilen zum Halten, Bewegen und Antreiben des Griffs (D) des Katheters (C) sich im Arm bewegt.

## Revendications

1. Manipulateur robotique pour manoeuvrer à distance des cathéters orientables (C) dans le système cardiovasculaire humain, de type à soumettre le cathéter, en soutenant le manche (D) et en raidissant sa portion extérieure au moyen d'un guide télescopique (E), à des mouvements avant et arrière axiaux, à une rotation axiale dans les deux directions, et aux mouvements de direction nécessaires pour permettre à l'extrémité du cathéter d'être commandée à travers les torsions et tours du système cardiovasculaire du patient (P) et atteindre un point prédéterminé dans ce système, en toute sécurité, d'une manière pouvant être répétée et contrôlée, le manche (D) était pourvu d'un curseur (F) adapté pour la commande de direction du cathéter, comprenant :
a) une structure (1) en mesure d'être fixée à des dispositifs de support réglables (H) avec une orientation et un positionnement corrects par rapport au patient ;
b) un bras (13) fixé en saillie et d'une manière rotative autour de l'arbre (4) de ladite structure (1), les dispositifs de réalisation de ladite rotation étant logés dans ladite structure ;
c) au minimum un chariot (18') étant monté dans ledit bras (13) de sorte qu'il puisse se déplacer dans le sens longitudinal, dans au minimum un guide approprié (16), le chariot présentant un dispositif de serrage intermédiaire (31-36) pour soutenir le manche (D) du cathéter (C) qui est aligné axialement sur ledit axe de rotation pour conférer au cathéter le mouvement de rotation axiale désiré, ledit manche (D) étant fermement serré sur ledit chariot (18') et aligné axialement sur ledit bras (4) de sorte que les rotations de cet arbre (4) soient transmises au cathéter (C) sans composants de translation indésirés ;
d) ledit chariot (18') pouvant être déplacé par des dispositifs d'entraînement placés dans le bras (13) pour produire le mouvement avant ou arrière du cathéter dans le système cardiovasculaire ;
e) un bloc de guidage coulissant (42) étant monté sur ledit chariot (18') avec un dispositif de préhension (44) opérant sur le curseur (F) prévu pour la commande de direction du cathéter, ce block de guidage coulissant étant actionné par des dispositifs d'entraînement associés au chariot (18') ;
f) où l'arbre de rotation (4) qui porte ledit bras (13) est de type creux et jouxte, à une extrémité de fin, un collecteur rotatif (12) dont le stator est fixé dans ladite structure (1) du manipulateur, tandis que l'autre extrémité de fin dudit arbre creux jouxte un canal (14) formé dans la base (113) dudit bras (13), à travers lequel passent tous les câbles électriques (57, 58) raccordés à tous les composants électroniques et électriques pourvus dans le bras, ces câbles étant raccordés par des dispositifs dudit collecteur auxdits câbles fixés qui sont également en partie raccordés aux différents composants électriques placés dans la structure pour la rotation dudit bras (13).

2. Manipulateur robotique selon la revendication 1, **caractérisé en ce qu'**il comprend un chariot avant (18') et un chariot arrière (18), les dispositifs d'entraînement qui confèrent les mouvements longitudinaux en avant et en arrière audit chariot (18') agissant sur le chariot arrière (18) qui est relié au chariot avant (18') en raccordant les dispositifs (26, 28) qui comprennent des dispositifs de préférence réglables et résistants (27) qui confèrent une pré-charge correcte d'un capteur de force (29) fixé au chariot avant (18'), ce capteur générant un signal électrique proportionnel à la force d'appui à laquelle le cathéter (C) est soumis quand il est déplacé en avant par le manipulateur dans le système cardiovasculaire humain.

3. Manipulateur robotique selon la revendication 2, **caractérisé en ce que** lesdits dispositifs de préférence réglables et résistants comprennent un ressort (27) et **en ce que** les dispositifs d'arrêt (30) sont prévus pour conférer le couplage mutuel entre lesdits chariots (18, 18') pendant le déplacement en arrière, de sorte à empêcher de comprimer ledit ressort (27) pour pré-charger le capteur de force (29).

4. Manipulateur robotique selon l'une ou plusieurs revendications parmi les revendications susmentionnées, **caractérisé en ce que** les dispositifs d'entraînement pour le mouvement longitudinal desdits deux chariots (18, 18') et dudit bloc de guidage coulissant (42) avec la commande de direction comprennent deux chaînes cinématiques, où, de préférence, des micro-commutateurs de fin de course (54, 54', 53, 53') actionnent également des unités de vis et d'écrous (21-20, 40-41) et des unités de motoréducteurs réversibles (22, 39) avec des encodeurs respectifs, des encodeurs correspondants (24, 52) étant pourvus directement sur les vis elles-mêmes, à partir desquels les signaux électriques sont engendrés en combinaison avec ceux provenant des encodeurs desdites unités d'entraînement de manière à identifier tout mauvais fonctionnement des deux chaînes cinématiques.

5. Manipulateur robotique selon l'une ou plusieurs revendications parmi les revendications susmentionnées, **caractérisé en ce que** l'arbre (13) qui porte le manche du cathéter est entraîné en rotation par une chaîne cinématique comprenant une unité de motoréducteurs réversibles (7) avec un encodeur, qui est fixé dans ladite structure (1) du manipulateur et qui, au moyen d'un pignon (6), entraîne une couronne (5) qui est verrouillée sur l'arbre (4) qui supporte de façon rotative ledit bras, cette couronne (5) étant prévue pour s'engager avec un deuxième pignon (9) qui entraîne un encodeur supplémentaire (10), dont le signal électrique est engendré en combinaison avec celui de l'encodeur dudit moteur (7) de sorte à identifier tout mauvais fonctionnement de cette chaîne cinématique qui comprend également des dispositifs (37) pour produire un signal électrique correspondant à une position de fin de course ou à une position zéro de cette chaîne cinématique.

6. Manipulateur robotique selon l'une ou plusieurs revendications parmi les revendications susmentionnées, **caractérisé en ce qu'**il comprend une pince d'auto-centrage résistante (31-36) pour soutenir le manche (D) du cathéter tout en permettant un montage et un démontage facile et rapide.

7. Manipulateur robotique selon l'une ou plusieurs revendications parmi les revendications susmentionnées, **caractérisé en ce qu'**il comprend des fourreaux (48, 49) qui sont en partie fixés et en partie mobiles et **en ce que** le bras (13) comprend une partie creuse, lesdits fourreaux étant en mesure de garder la partie creuse du bras (13) constamment fermée de l'extérieur quand ledit chariot composite (18, 18'), avec les parties associées pour soutenir, déplacer et entraîner le manche (D) du cathéter (C), se déplace dans le bras.
